# EUROPEAN PATENT SPECIFICATION

(11) **EP 2 615 657 B1**
(45) Date of publication and mention of the grant of the patent: **21.12.2016**
(21) Application number: 11823548.0
(22) Date of filing: 06.09.2011
(51) Int. Cl.: H01L 51/30, C07D 495/04, C08K 5/45, C08L 65/00, H01L 21/336, H01L 29/786, H01L 51/05, H01L 51/40

(54) **ORGANIC SEMICONDUCTOR MATERIAL, ORGANIC SEMICONDUCTOR COMPOSITION, ORGANIC THIN FILM, FIELD-EFFECT TRANSISTOR, AND MANUFACTURING METHOD THEREFOR**
ORGANISCHES HALBLEITERMATERIAL, ORGANISCHE HALBLEITERZUSAMMENSETZUNG, ORGANISCHE DÜNNSCHICHT, FELDEFFEKTTRANSISTOR UND HERSTELLUNGSVERFAHREN DAFÜR
MATÉRIAU SEMI-CONDUCTEUR ORGANIQUE, COMPOSITION SEMI-CONDUCTRICE ORGANIQUE, FILM MINCE ORGANIQUE, TRANSISTOR À EFFET DE CHAMP, ET PROCÉDÉ DE FABRICATION ASSOCIÉ

(30) Priority: 07.09.2010 JP 2010199956
(43) Date of publication of application: 17.07.2013
(73) Proprietor: Nippon Kayaku Kabushiki Kaisha, Tokyo 102-8172 (JP)
(72) Inventor: SADAMITSU, Yuichi, Tokyo 115-8588 (JP)
(74) Representative: Gille Hrabal
(86) International application number: PCT/JP2011/070214
(87) International publication number: WO 2012/033073

(56) References cited:
- WO-A1-03/052841
- WO-A1-2009/122956
- JP-A- 61 168 625
- JP-A- 2007 063 317
- JP-A- 2009 096 989
- JP-A- 2009 267 372
- H. EBATA ET AL.: 'Highly Soluble [1]Benzothieno [3,2-b]benzothiophene (BTBT) Derivatives for High-Performance, Solution-Processed Organic Field-Effect Transistors' JOURNAL OF THE AMERICAN CHEMICAL SOCIETY vol. 129, 29 November 2007, pages 15732 - 15733, XP009147953
- T. MINARI ET AL.: 'Surface selective deposition of molecular semiconductors for solution-based integration of organic field-effect transistors' APPLIED PHYSICS LETTERS vol. 94, 06 March 2009, pages 093307-1 - 093307-3, XP012119336

## Description

### Technical Field

The present invention relates to an organic semiconductor material, an organic semiconductor composition, an organic thin film, a transistor formed by applying or printing an organic semiconductor material, and a method of producing the transistor. More specifically, the present invention relates to (i) a field-effect transistor that has a specific structure and that is formed with use of a semiconductor made from a composition prepared from an organic heterocyclic compound and a specific polymer material and (ii) a method of producing the field-effect transistor.

### Background Art

In general, a field-effect transistor is structured such that (i) a source electrode and a drain electrode are provided on a semiconductor material on a substrate and (ii) a gate electrode etc. is provided on the source and drain electrodes via an insulation layer. Today, inorganic semiconductor materials such as silicon are used in field-effect transistors. In particular, a thin film transistor formed from amorphous silicon, which is provided on a substrate such as a glass substrate, is used in a display etc. The thin film transistor is widely used as a logical circuit element of an integrated circuit and as a switching element etc. Further, recently, using an oxide semiconductor as a semiconductor material is being actively studied. However, in a case of producing a field-effective transistor by using such an inorganic semiconductor material, it is necessary to subject the field-effect transistor to high temperatures or vacuum during the production. Therefore, it is not possible to use a substrate made from a film or plastic etc. which is less resistant to heat, and expensive equipment and a lot of energy are required for the production of the field-effect transistor. This results in very high costs, and such a field-effect transistor is used only for very limited applications.

On the other hand, there has been development of a field-effect transistor using an organic semiconductor material. Such a field-effect transistor can be produced without high-temperature treatments. Being able to use the organic semiconductor material will allow production with low-temperature processes, and thus use of various substrate materials will become available. This makes it possible to produce a more flexible, lightweight field-effect transistor less prone to breakages. Further, by producing the field-effect transistor by applying a solution containing the organic semiconductor material or by a printing method such as ink-jet printing, it may be possible to produce a large-area field-effect transistor at low cost.

However, most of the organic compounds that have conventionally been used in the organic semiconductor material are poorly soluble in organic solvents, and thus economical methods such as an application printing method are not applicable. Therefore, generally a relatively high-cost method such as a vacuum deposition method has been used to allow a thin film to form on a substrate of a semiconductor. In recent years, it has become possible to obtain a device having a relatively high carrier mobility, by producing a field-effect transistor by forming a film by an application method with use of an organic semiconductor material that is soluble in organic solvents. However, as of today, a method of producing a highly durable field-effect transistor that includes an organic semiconductor and has a high mobility by an application printing process has not been put into practical use. How to produce a transistor with improved properties has been studied actively even today.

Patent Literature 1 discloses a field-effect transistor formed with use of (i) an aryl derivative of benzoseleno [3,2-b][1] benzoselenophene (a compound represented by the formula (1) wherein a sulfur atom is replaced by a selenium atom and R₁ and R₂ each represent a hydrogen atom) and (ii) an aryl derivative of benzothieno [3,2-b][1] benzothiophene (a compound represented by the formula (1) wherein R₁ and R₂ each represent a hydrogen atom).

Patent Literature 2 discloses a field-effect transistor formed with use of an alkyl derivative of benzoseleno [3,2-b][1] benzoselenophene and an alkyl derivative of benzothieno [3,2-b][1] benzothiophene.

Patent Literature 3 discloses a field-effect transistor formed with use of a mixture of an alkyl derivative of benzothieno [3,2-b][1] benzothiophene and a polymer having a specific solubility parameter.

Patent Literature 4 discloses a field-effect transistor formed with use of a composition containing an alkyl derivative of benzothieno [3,2-b][1] benzothiophene and a polymer material.

Patent Literature 5 discloses a field-effect transistor formed with use of for example a composition obtained by mixing (i) a pentacene derivative made soluble in organic solvents by introducing thereto a specific substituent and (ii) a polymer.

Non Patent Literature 1 discloses a field-effect transistor formed with use of an alkyl derivative of benzothieno [3,2-b][1] benzothiophene.

Non Patent Literature 2 discloses a field-effect transistor formed with use of an alkyl derivative of benzothieno [3,2-b][1] benzothiophene by a surface selective deposition method.

EP 2 264 804 corresponding to WO 2009/122956 discloses an organic semiconductor composition comprising benzothienobenzothiophene and polytriarylamine compounds. However, the aryl groups of the polytriarylamine are not necessarily phenyl groups as in compound of the formula (2) of the present invention.

WO 03/052841 discloses an insulating material for an organic FET and mentions that a polydiphenyl(2,4-dimethylphenyl)amine is used as a semiconductor material in field effect transistors.

### Citation List

### Patent Literatures

Patent Literature 1 Pamphlet of International Publication No. WO2006/077888
Patent Literature 2 Pamphlet of International Publication No. WO2008/047896
Patent Literature 3 Japanese Patent Application Publication, Tokukai, No. 2009-267372 A
Patent Literature 4 Japanese Patent Application Publication, Tokukai, No. 2009-283786 A
Patent Literature 5 Japanese Translation of PCT Patent Application, Tokuhyo, No. 2009-524226 A
Patent Literature 6 Pamphlet of International Publication No. WO1999/32537
Patent Literature 7 Pamphlet of International Publication No. WO1998/6773

### Non Patent Literatures

Non Patent Literature 1 J. Am. Chem. Soc. 2007, 129, 15732.
Non Patent Literature 2 Applied Physics Letters, 94, 93307, 2009.
Non Patent Literature 3 J. Org. Chem. 1986, 51, 2627

### Summary of Invention

### Technical Problem

An object of the present invention is to provide a practical field-effect transistor having excellent suitability for printing that enables formation of a highly uniform thin film, and further having excellent semiconducting properties such as carrier mobility, hysteresis and threshold stability.

### Solution to Problem

The inventors of the present invention have diligently worked to attain the above object, and found that, by forming a field-effect transistor with use of a semiconductor made from a composition obtained by mixing a specific organic heterocyclic compound and a specific polymer to an organic solvent, it is possible to provide a practical field-effect transistor having excellent suitability for printing that enables formation of a highly uniform thin film and further having excellent semiconducting properties such as carrier mobility, hysteresis and threshold stability. Then, the inventors have completed the present invention.

Specifically, the present invention relates to <1> an organic semiconductor material containing a compound represented by the formula (1) and a compound represented by the formula (2): 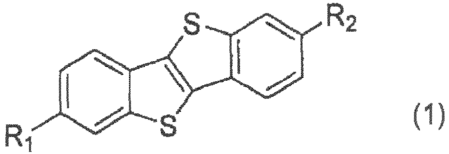 wherein R₁ and R₂ independently represent an unsubstituted or halogen-substituted C1-C36 aliphatic hydrocarbon group; and 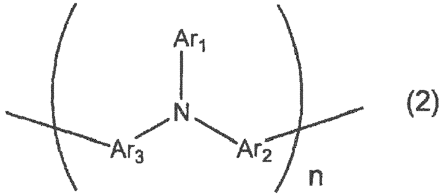 wherein Ar₁, Ar₂ and Ar₃ are defined as in claim 1, and n is an integer of 6 or greater.

### Advantageous Effects of Invention

By forming a field-effect transistor with use of an organic semiconductor material containing a compound represented by the formula (1) and a compound represented by the formula (2), it is possible to provide a practical field-effect transistor having excellent suitability for printing that enables formation of a highly uniform thin film and further having excellent semiconducting properties such as carrier mobility, hysteresis and threshold stability.

### Brief Description of Drawings

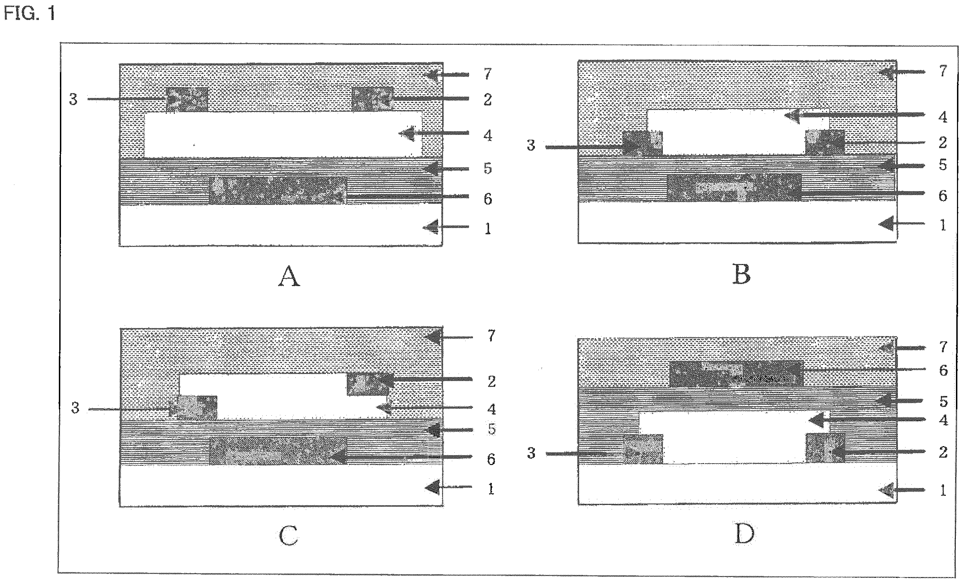

Fig. 1
   A to D of Fig. 1 are views schematically illustrating examples of a structure of a field-effect transistor of the present invention.
Fig. 2

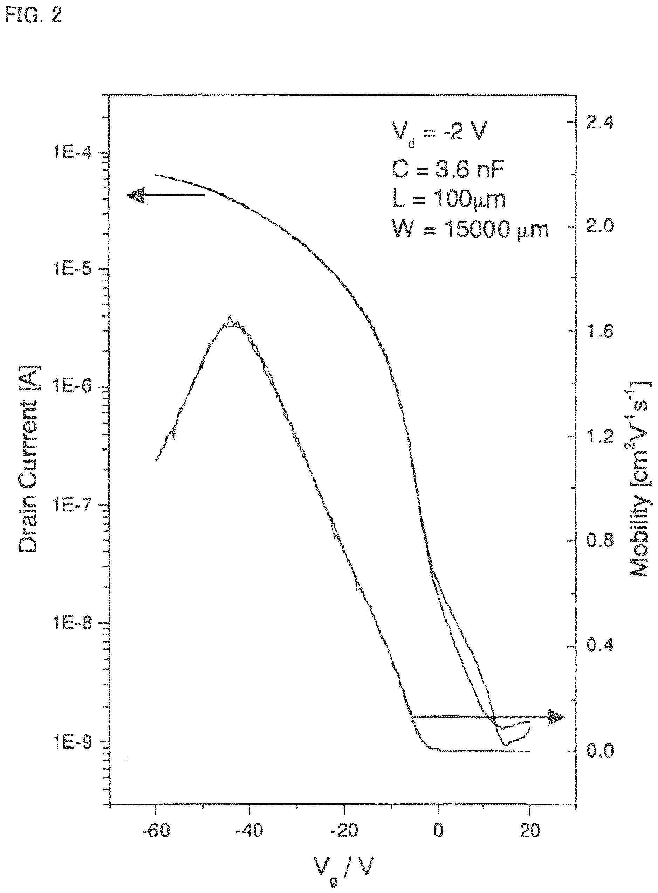

   Fig. 2 is a graph showing characteristics of a field-effect transistor of the present invention.
Fig. 3

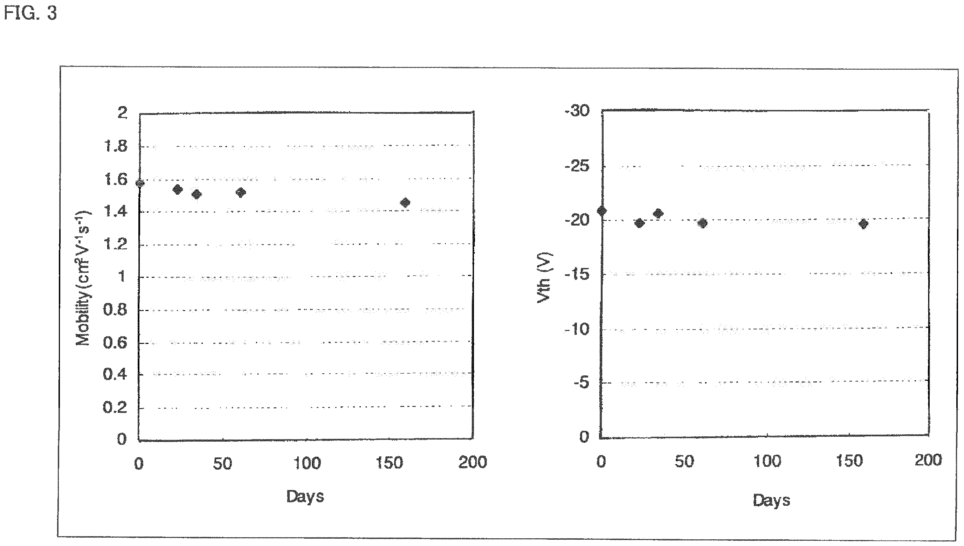

   Fig. 3 is a graph showing how a field-effect transistor of the present invention is stable in atmosphere.

### Description of Embodiments

The following description discusses the present invention in detail. The present invention relates to an organic semiconductor material containing a specific organic heterocyclic compound and a specific polymer, an organic thin film, a field-effect transistor formed with use of the organic semiconductor material and the organic thin film, and a method of producing the field-effect transistor.

First, the following describes the compound represented by the formula (1). In the formula (1), R₁ and R₂ independently represent an unsubstituted or halogen-substituted C 1-C36 aliphatic hydrocarbon group. The aliphatic hydrocarbon group is a saturated or unsaturated, linear, branched or cyclic aliphatic hydrocarbon group. The aliphatic hydrocarbon group is preferably a linear or branched aliphatic hydrocarbon group, and further preferably a linear aliphatic hydrocarbon group. The aliphatic hydrocarbon group is normally a C1-C36 aliphatic hydrocarbon group, preferably a C2-C24 aliphatic hydrocarbon group, more preferably a C4-C20 aliphatic hydrocarbon group, and further preferably a C6-C 12 aliphatic hydrocarbon group.

Specific examples of a saturated linear or branched aliphatic hydrocarbon group include methyl, ethyl, propyl, iso-propyl, n-butyl, iso-butyl, t-butyl, n-pentyl, iso-pentyl, t-pentyl, sec-pentyl, n-hexyl, iso-hexyl, n-heptyl, sec-heptyl, n-octyl, n-nonyl, sec-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosyl, docosyl, n-pentacosyl, n-octacosyl, 5-(n-pentyl) decyl, heneicosyl, tricosyl, tetracosyl, hexacosyl, heptacosyl, nonacosyl, n-triacontyl, dotriacontyl, and hexatriacontyl.

Specific examples of a saturated cyclic aliphatic hydrocarbon group include cyclohexyl, cyclopentyl, adamantyl, and norbornyl.

Specific examples of an unsaturated linear or branched aliphatic hydrocarbon group include vinyl, aryl, eicosa dienyl, 11,14-eicosa dienyl, geranyl (trance-3,7-dimethyl-2,6-octadien-1-yl), farnesyl (trance, trance-3,7,11-trimethyl-2,6,10-dodecatrien-1-yl) , 4-pentenyl, 1-propynyl, 1-hexynyl, 1-octynyl, 1-decynyl, 1-undecynyl, 1-dodecynyl, 1-tetradecynyl, 1-hexadecynyl, and 1-nonadecynyl.

Out of linear, branched and cyclic aliphatic hydrocarbon groups, preferred is a linear or branched aliphatic hydrocarbon group, and further preferred is a linear aliphatic hydrocarbon group.

Examples of a saturated or unsaturated aliphatic hydrocarbon group include: a saturated alkyl group; an alkenyl group including a carbon-carbon double bond; and an alkynyl group including a carbon-carbon triple bond. An alkyl group or an alkynyl group is more preferable, and an alkyl group is further preferable. An aliphatic hydrocarbon residue encompasses a combination of such a saturated aliphatic hydrocarbon group and an unsaturated aliphatic hydrocarbon group. That is, aliphatic hydrocarbon groups each containing both a carbon-carbon double bond and a carbon-carbon triple bond are all regarded as the aliphatic hydrocarbon residue.

A halogen-substituted aliphatic hydrocarbon group means an aliphatic hydrocarbon group in which any position(s) is/are substituted by a halogen atom(s) of any kind. Examples of a halogen atom include a fluorine atom, a chlorine atom, a bromine atom and an iodine atom. A fluorine atom, a chlorine atom and a bromine atom are preferable, and a fluorine atom and a bromine atom are further preferable. Specific examples of a halogen-substituted aliphatic hydrocarbon group include chloromethyl, bromomethyl, trifluoromethyl, pentafluoroethyl, n-perfluoro propyl, n-perfluoro butyl, n-perfluoro pentyl, n-perfluoro octyl, n-perfluoro decyl, n-(dodecafluoro)-6-iodohexyl, 2,2,3,3,3-pentafluoropropyl, and 2,2,3,3-tetrafluoropropyl.

The compound represented by the formula (1) can be synthesized by a known method described in for example Non Patent Literature 1. The compound can be obtained also by the method described in Patent Literature 2.

How to purify the compound represented by the formula (1) is not particularly limited. The compound can be purified by a known method such as recrystallization, column chromatography or vacuum sublimation purification. Further, these methods can be used in combination as needed.

The following Table 1 shows specific examples of the compound represented by the formula (1).

**Table 1**

| Compound No. | R1 | R2 |
|---|---|---|
| 1 | CH₃ | CH₃ |
| 2 | C₂H₅ | C₂H₅ |
| 3 | n-C₃H₇ | n-C3H₇ |
| 4 | t-C₄H₉ | t-C₄H₉ |
| 5 | n-C₅H₁₁ | n-C₅H₁₁ |
| 6 | sec-C₅H₁₁ | sec-C₅H₁₁ |
| 7 | n-C₆H₁₃ | n-C₆H₁₃ |
| 8 | iso-C₅H₁₃ | iso-C₆H₁₃ |
| 9 | n-C₇H₁₅ | n-C₇H₁₅ |
| 10 | sec-C₇H₁₅ | sec-C₇H₁₅ |
| 11 | n-C₈H₁₇ | n-C₈H₁₇ |
| 12 | n-C₉H₁₉ | n-C₉H₁₉ |
| 13 | n-C₁₀H₂₁ | n-C₁₀H₂₁ |
| 14 | n-C₁₁H₂₃ | n-C₁₁H₂₃ |
| 15 | n-C₁₂H₂₅ | n-C₁₂H₂₅ |
| 16 | n-C₁₃H₂₇ | n-C₁₃H₂₇ |
| 17 | n-C₁₄H₂₉ | n-C₁₄H₂₉ |
| 18 | n-C₁₅H₃₁ | n-C₁₅H₃₁ |
| 19 | n-C₁₆H₃₃ | n-C₁₆H₃₃ |
| 20 | n-C₁₇H₃₅ | n-C₁₇H₃₅ |
| 21 | n-C₁₈H₃₇ | n-C₁₈H₃₇ |
| 22 | n-C₂₀H₄₁ | n-C₂₀H₄₁ |
| 23 | n-C₂₂H₄₅ | n-C₂₂H₄₅ |
| 24 | n-C₂₄H₄₉ | n-C₂₄H₄₉ |
| 25 | n-C₃₀H₆₁ | n-C₃₀H₅₁ |
| 26 | n-C₃₆H₇₃ | n-C₃₆H₇₃ |
| 27 | C₅H₉(C₅H₁₁)₂ | C₅H₉(C₅H₁₁)₂ |
| 28 | n-C₉H₁₉ | sec-C₉H₁₉ |
| 29 | n-C₆H₁₃ | sec-C₉H₁₉ |
| 30 | n-C₈H₁₇ | n-C₁₀H₂₁ |
| 31 | n-C₈H₁₇ | n-C₁₂H₂₅ |
| 32 | n-C₈H₁₆Cl | n-C₈H₁₆Cl |
| 33 | n-C₈H₁₆Br | n-C₈H₁₆Br |
| 34 | CH₂Cl | CH₂Cl |
| 35 | C₃F₇ | C₃F₇ |
| 36 | C₄F₉ | C₄F₉ |
| 37 | C₈F₁₇ | C₈F₁₇ |
| 38 | C₁₀F₂₁ | C₁₀F₂₁ |
| 39 | -CH₂C₂F₅ | -CH₂C₂F₅ |
| 40 | -CH₂CF₂CHF₂ | -CH₂CF₂CHF₂ |
| 41 | -CH=CH₂ | -CH=CH₂ |
| 42 | -CH₂CH=CH₂ | -OH₂CH=CH₂ |
| 43 | -C₄H₈CH=CH₂ | -C₄H₈CH=CH₂ |
| 44 | -C≡CC₆H₁₃ | -C≡CC₆H13 |
| 45 | -O≡CC₈H₁₇ | -C≡CC₈H₁₇ |
| 46 | -C≡CC₁₀H₂₁ | -C≡CC₁₀H₂₁ |
| 47 | -C≡CC₁₂H₂₅ | -C≡CC₁₂H₂₅ |
| 48 | -C≡CC₆H₁₃ | -C≡CC₆H₁₃ |
| 49 | cycloC₅H₉ | cycloC₅H₉ |
| 50 | cycloC₆H₁₁ | cycloC₆H₁₁ |

Next, the following describes the compound represented by the formula (2). In the formula (2), Ar₁, Ar₂ and Ar₃ are defined as in claim 1, and n is an integer of 6 or greater. Ar₁, Ar₂ and Ar₃ are each a phenyl group. In a case where Ar₁, Ar2 and Ar₃ have a substituent(s), the position of the substituent is not particularly limited. The substituent on the aryl group can be a hydrogen atom (except for Ar¹), a halogen atom, a C1-C12 alkyl group, a C1-C12 alkoxyl group, a C1-C12 halogeno alkyl group, a C1-C12 halogeno alkoxyl group, and/or a cyano group. Specific examples of a C1-C12 alkyl group include methyl, ethyl, propyl, iso-propyl, n-butyl, iso-butyl, t-butyl, n-pentyl, iso-pentyl, t-pentyl, sec-pentyl, n-hexyl, iso-hexyl, n-heptyl, sec-heptyl, n-octyl, n-nonyl, sec-nonyl, n-decyl, n-undecyl, and n-dodecyl. Further, examples of a C1-C12 alkoxyl group include methoxy, ethoxy, propoxy, and butoxy. Specific examples of a C1-C12 halogeno alkyl group include: chloro-substituted alkyl such as chloromethyl and trichloromethyl; and fluoro-substituted alkyl such as trifluoromethyl, trifluoroethyl and pentafluoroethyl. Examples of a C1-C12 halogeno alkoxyl group include trifluoromethoxy and pentafluoroethoxy. Preferred is a phenyl group substituted with, out of the above substituents, particularly a halogen atom, a C1-C4 alkyl group, a C1-C4 halogeno alkyl group, a C 1-C4 alkoxyl group, a C1-C4 halogeno alkoxyl group, or a cyano group. Ar₁ is preferably a phenyl substituted with a halogen atom, a C1-C4 alkyl group, a C1-C4 halogeno alkyl group, a C1-C4 alkoxyl group, a C1-C4 halogeno alkoxyl group or a cyano group. Ar₂ and Ar₃ are preferably unsubstituted phenyl. Further, the structure of the following formula (3) in which 2-, 4- and 6-positions of Ar₁ are substituted is preferable. In the formula (3), at least one of R₃, R₄ and R₅ is preferably a halogen atom, a C1-C4 alkyl group, a C1-C4 alkoxyl group, a C1-C4 halogeno alkyl group, a C 1-C4 halogeno alkoxyl group or a cyano group; and the other(s) is preferably a hydrogen atom, a halogen atom, a C1-C4 alkyl group, a C1-C4 alkoxyl group, a C1-C4 halogeno alkyl group, a C1-C4 halogeno alkoxyl group or a cyano group; and, in particular, at least one of R₃, R₄ and R₅ is preferably a methyl group, a trifluoromethyl group, a methoxy group, a trifluoromethoxy group or a fluoro group. n is an integer of 6 or greater, but is preferably at least 15 or greater. The molecular weight of the compound represented by the formula (3) is 5000 or greater. Note that the compound represented by the formula (2) can be synthesized by a known method described in for example Patent Literature 6, Patent Literature 7 or Non Patent Literature 3.

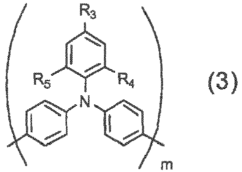

An organic semiconductor material of the present invention contains at least a compound represented by the formula (1) and a compound represented by the formula (2). The organic semiconductor material can contain (i) one compound represented by the formula (1) and one compound represented by the formula (2) or (ii) a mixture of several derivatives of one of or both of the compounds represented by the respective formulae (1) and (2). The organic semiconductor material of the present invention contains the compound represented by the formula (1) in an amount of preferably 10 to 99 % by mass, more preferably 30 to 95 % by mass, and further preferably 50 to 85 % by mass relative to the total amount of the organic semiconductor material. On the other hand, the organic semiconductor material of the present invention contains the compound represented by the formula (2) in an amount of preferably 1 to 90 % by mass, more preferably 5 to 70 % by mass, and further preferably 15 to 50 % by mass relative to the total amount of the organic semiconductor material.

To the organic semiconductor material of the present invention, another organic semiconductor material or an additive of various kinds can be mixed as needed to improve the characteristics of a field-effect transistor or to impart other characteristics to the field-effect transistor, provided that the effects of the present invention are not impaired. Examples of the additive include carrier generating agents, electrically conducting substances, viscosity modifying agents, surface tension modifying agents, leveling agents, penetrating agents, rheology modifying agents, aligning agents, and dispersants.

The organic semiconductor material of the present invention can contain such an additive in an amount of 0 to 30 % by mass, preferably 0 to 20 % by mass, and further preferably not more than 10 % by mass, relative to the total amount of the organic semiconductor material.

Next, in order for the organic semiconductor material to be usable in an application printing process, it is preferable that the organic semiconductor material of the present invention be dissolved or dispersed in an organic solvent to form an organic semiconductor composition. A solvent that can be used is not particularly limited provided that it is possible to form a film of a compound on a substrate, but an organic solvent is preferable. A single organic solvent can be used or a mixture of a plurality of organic solvents can be used. Specific examples of the organic solvent include: aromatic hydrocarbon solvents such as toluene, xylene, mesitylene, ethyl benzene, diethylbenzene, triethylbenzene, tetrahydronaphthalene, decaline, and cyclohexylbenzene; hydrocarbon solvents such as hexane, heptane, cyclohexane, octane and decane; alcohol solvents such as methanol, ethanol, isopropyl alcohol, and butanol; fluoroalcohol solvents such as octafluoropentanol and pentafluoropropanol; ester solvents such as ethyl acetate, butyl acetate, ethyl benzoate, and diethyl carbonate; ketone solvents such as acetone, methyl ethyl ketone, methyl isobutyl ketone, cyclopentanone, and cyclohexanone; amide solvents such as dimethylformamide, dimethylacetamide, and N-methylpyrrolidone; and ether solvents such as tetrahydrofuran and diisobutyl ether. Note however that, in view of an actual application printing process, it is necessary to take into consideration the safety of the solvent and composition stability during the storage and production. Therefore, it is preferable that at least one organic solvent have a boiling point of 150°C or higher, and is further preferable to use at least one solvent having a boiling point of 180°C or higher. That is, the organic semiconductor composition in accordance with the present invention is preferably composed of a solution containing at least one organic solvent whose boiling point is 150°C or higher, and more preferably composed of a solution containing at least one organic solvent whose boiling point is 180°C or higher. Note that, in these solutions, solutes are uniformly dissolved in solvents.

A field-effect transistor (field effect transistor, hereinafter may be referred to as FET for short) of the present invention has two electrodes (source and drain electrodes) in contact with a semiconductor layer, and is configured such that an electric current passing between the two electrodes is controlled by a voltage applied to another electrode (called gate electrode) via a gate insulation film.

Fig. 1 shows several examples of a configuration of the field-effect transistor of the present invention. Note, however, that how to arrange the layers and electrodes can be selected as appropriate depending on the applications of the element.

The following description discusses constituents of the field-effect transistor of the present invention shown in Fig. 1. Note, however, that the field-effect transistor in accordance with the present invention is not limited to these constituents. Further note that, in Fig. 1, the constituents having the same name are assigned identical numbers.

A substrate 1 needs to hold each layer to be formed thereon so that the each layer does not become separated. For example, the substrate 1 can be made from: an insulation material such as a resin plate, a resin film, paper, glass, quartz, or ceramic; a substance obtained by coating, with an insulation layer, a conductive substrate made from metal or alloy; or a material obtained from a combination of a resin and an inorganic material etc. Out of these, a resin film is used in general. Examples of the resin film include: polyethylene terephthalate, polyethylene naphthalate, polyether sulfone, polyamide, polyimide, polycarbonate, cellulose triacetate, and polyether imide. Using a resin film or paper makes it possible to impart flexibility to a semiconductor element, and thus the semiconductor element becomes flexible, lightweight and more practical. The thickness of the substrate is normally 1 µm to 10 mm, and preferably 5 µm to 3 mm.

A source electrode 2, a drain electrode 3 and a gate electrode 6 are made from a material having electrical conductivity. Examples of a material for these electrodes include: metals such as platinum, gold, silver, aluminum, chromium, tungsten, tantalum, nickel, cobalt, copper, iron, lead, tin, titanium, indium, palladium, molybdenum, magnesium, calcium, barium, lithium, potassium, and sodium, and alloys containing these metals; electrically conductive oxides such as InO₂, ZnO₂, SnO₂, and ITO; electrically conductive polymers such as polyaniline, polypyrrole, polythiophene (e.g., PEDOT and PSS), polyacetylene, polyparaphenylenevinylene, and polydiacetylene; organic charge-transfer complexes such as BED-TTF; semiconductors such as silicon, germanium, and gallium arsenide; and carbon materials such as carbon black, fullerene, carbon nanotubes, and graphite. An electrically conductive polymer or a semiconductor can be subjected to doping. Examples of a dopant include: acids such as hydrochloric acid, sulfuric acid and sulfonic acid; Lewis acids such as PF₅, AsF₅, and FeCl₃; halogen atoms such as iodine; and a metal atom such as lithium, sodium and potassium. To reduce contact resistance of the electrodes, molybdenum oxide can be doped or a metal can be treated with thiol etc. Further, it is possible to use an electrically conductive composite material obtained by dispersing metal particles etc. of carbon black, gold, platinum, silver, copper or the like into the above materials. Wires connected to the electrodes 2, 3 and 6 can be formed from substantially the same materials as those for the electrodes. The thickness of each of the source, drain and gate electrodes 2, 3 and 6 depends on the materials, but is normally 0.1. nm to 100 µm, preferably 0.5 nm to 10 µm, and more preferably 1 nm to 5 µm.

A gate insulation layer 5 is made from an insulating material. Examples of the insulating material include:
polymers such as polyparaxylylene, polyacrylate, polymethyl methacrylate, polystyrene, polyvinylphenol, polyamide, polyimide, polycarbonate, polyester, polyvinyl alcohol, polyvinyl acetate, polyurethane, polysulfone, epoxy resin, and phenol resin, and copolymers of a combination thereof; oxides such as silicon dioxide, aluminum oxide, titanium oxide, and tantalum oxide; ferroelectric oxides such as SrTiO₃ and BaTiO₃; nitrides such as silicon nitride and aluminum nitride; sulfides; and dielectric materials such as fluoride; and polymers in which particles of these dielectric materials are dispersed. The thickness of the gate insulation layer 5 depends on the materials, but is normally 0.1 nm to 100 µm, preferably 0.5 nm to 50 µm, and more preferably 5 nm to 10 µm.

The organic semiconductor material contained in the semiconductor layer 4 contains at least the compound represented by the formula (1) and the compound represented by the formula (2). The organic semiconductor material can contain a mixture of a several derivatives of the compound represented by the formula (1) and/or the compound represented by the formula (2). The organic semiconductor material is contained in an amount of not less than 50 % by mass, preferably not less than 80 % by mass, and further preferably not less than 95 % by mass relative to the total amount of the semiconductor layer 4. Note here that another organic semiconductor material or an additive of various kinds can be mixed as needed to improve the characteristics of a field-effect transistor or to impart other characteristics to the field-effect transistor. Further, the semiconductor layer 4 can consist of a plurality of layers. The film thickness of the semiconductor layer 4 is preferably as small as possible, provided that the necessary functions are not impaired. In field-effect transistors, the characteristics of a semiconductor element do not depend on the film thickness as long as the film thickness is equal to or larger than a predetermined film thickness. However, as the film thickness becomes large, a leakage current often increases. On the other hand, if the film thickness is too small, it becomes impossible to form a channel for electric charge. Therefore, an appropriate film thickness is necessary. The film thickness of the semiconductor layer necessary for a semiconductor to have necessary functions is normally 0.1 nm to 10 µm, preferably 0.5 nm to 5 µm, and more preferably 1 nm to 3 µm.

A material for a protection layer 7 is not particularly limited. Preferable examples of the material include: films made from various resins such as epoxy resin, acrylic resin such as polymethyl methacrylate, polyurethane, polyimide, polyvinyl alcohol, fluororesin and polyolefin; and inorganic oxide films and nitride films made from dielectric materials such as silicon oxide, aluminum oxide, and silicon nitride. In particular, a resin (polymer) having a low oxygen transmission rate and low water absorption rate is preferable. Alternatively, the protection layer 7 can be made from a protection material developed for an organic EL display. The film thickness of the protection layer can be determined as appropriate depending on the purpose of the protection layer, but is normally 100 nm to 1 mm. Providing a protection layer makes it possible to reduce the effects of outside air such as humidity, and is advantageous in that stable electrical characteristics can be achieved, e.g., ON-OFF ratio of a device can be improved.

The field-effect transistor of the present invention can exhibit excellent suitability for printing by subjecting a surface of the substrate to a cleaning treatment such as: acid treatment using hydrochloric acid, sulfuric acid and/or acetic acid etc.; alkali treatment using sodium hydroxide, potassium hydroxide, calcium hydroxide and/or ammonia etc.; ozone treatment; fluorination treatment; plasma treatment using oxygen and/or argon etc.; treatment of forming a Langmuir-Blodgett film; treatment of forming a thin film of other insulating material or semiconductor; mechanical treatment; and electrical treatment such as corona discharge. Alternatively, another layer can be provided as needed between the foregoing layers or on an outer surface of a semiconductor element. Further, by preliminary carrying out a surface treatment of a substrate on which a semiconductor layer is to be stacked or of an insulation layer, it is possible to control molecular orientation and crystallization of a boundary face between the substrate or the electrodes etc. and the semiconductor layer to be formed thereafter. This reduces the trapping positions on an electrode interface and the insulation layer, and thus improves properties such as carrier mobility. Further, since hydrophilic-hydrophobic balance on the surface of the substrate is controlled, it is possible to improve quality of the film formed on the substrate and wettability against the substrate. This makes it possible to further improve uniformity of the device. Examples of such a treatment of the substrate include a silane coupling treatment using phenylethyl trichlorosilane etc., a thiol treatment, and a rubbing treatment using fiber.

Each of the layers in the present invention can be formed by for example an appropriate method selected from a vacuum deposition method, a sputtering method, an application method, a printing method, a sol-gel method and the like. In view of productivity, an application method or a printing method such as ink-jet printing is preferable.

The following description discusses, on the basis of the examples shown in Fig. 1, a method of producing a field-effect transistor of the present invention.

### (Substrate and treatment of substrate)

A field-effect transistor of the present invention is formed by providing necessary electrodes and various layers on the foregoing substrate 1 (refer to Fig. 1). The substrate 1 can be subjected to the foregoing surface treatment etc. The thickness of the substrate is preferably as small as possible provided that the necessary functions are not impaired. The thickness of the substrate depends on the material, but is normally 1 µm to 10 mm, and is preferably 5 µm to 3 mm.

### (Formation of source electrode and drain electrode)

The source electrode 2 and the drain electrode 3, which are made from the foregoing electrode material etc., are formed on the substrate 1. The source electrode 2 and the drain electrode 3 can be made from the same material or respective different materials. The electrodes are formed by for example a vacuum deposition method, a sputtering method, an application method, a thermal transfer method, a printing method, a sol-gel method or the like. At the time when or after a film is formed, it is preferable that the film be patterned as needed so that a desired shape is achieved. The patterning can be carried out by various methods, and is carried out by for example photolithography which is a combination of pattering and etching of for example a photoresist. Alternatively, the patterning can be carried out by: a printing method such as an ink-jet printing, a screen printing, an offset printing or a anastatic printing; a soft lithography method such as a microcontact printing; and a combination of two or more of these methods. The film thickness of each of the source and drain electrodes 2 and 3 depends on the material, but is normally 1 nm to 100 µm, preferably 0.5 nm to 10 µm, and more preferably 1 nm to 5 µm. The source electrode 2 and the drain electrode 3 can be the same or different in film thickness.

### (Formation of semiconductor layer)

A semiconductor layer is made from the foregoing organic semiconductor materials. The semiconductor materials are dissolved or dispersed in a solvent to form an organic semiconductor composition, which is used in an application printing process to form the semiconductor layer.

The application printing process is a method of producing a semiconductor layer, by which it is possible to easily form a semiconductor layer having excellent semiconducting properties. The application printing process is a method of (i) applying an organic semiconductor composition (to for example a substrate) obtained by preliminarily dissolving a solvent-soluble semiconductor material, e.g., a compound represented by the formula (1) and a compound represented by the formula (2) of the present invention, in an organic solvent and thereafter (ii) drying the organic semiconductor composition. Such a method of production by application, i.e., the application printing process, does not require vacuum or high-temperature environments during the production of a device, and is thus industrially advantageous because the process enables a low cost production of large-area field-effect transistors. For this reason, the application printing process is particularly preferable among various methods of producing semiconductor layers.

Specifically, the compound represented by the formula (1) and the compound represented by the formula (2) are dissolved or dispersed in a solvent. In this way, an organic semiconductor composition of the present invention is prepared. The compound represented by the formula (1) and the compound represented by the formula (2) can be simultaneously dissolved or dispersed or can be separately dissolved or dispersed, and thereafter mixed together to form the organic semiconductor composition. The concentration of the compounds represented by the respective formulae (1) and (2) or a plurality of the compounds in the composition depends on the type of a solvent and the film thickness of a semiconductor layer to be formed, but is normally 0.001 to 50 % by mass, preferably 0.01 to 20 % by mass, and particularly preferably not less than 0.5 % by mass but not more than 5 % by mass relative to the total amount of the organic semiconductor composition. An additive or another semiconductor material can be mixed to improve film-forming property of the semiconductor layer, improve characteristics of a field-effect transistor, and impart other characteristics to the field-effect transistor.

In order to prepare the organic semiconductor composition, it is necessary to dissolve or disperse the foregoing organic semiconductor material etc. in the foregoing solvent. This can be achieved by a thermal dissolution treatment as needed. Further, an obtained composition of the organic semiconductor material can be filtered so that impurities etc. are removed. Such a composition from which the impurities have been removed provides improved film-forming property of a semiconductor layer when applied to a substrate. Accordingly, the organic semiconductor composition containing the compounds represented by the respective formulae (1) and (2) is suitably used.

The organic semiconductor composition thus prepared is applied to a substrate (exposed parts of the source electrode and the drain electrodes). The organic semiconductor composition can be applied by: a coating method such as casting, spin coating, dip coating, blade coating, wire-bar coating or spray coating; a printing method such as ink-jet printing, screen printing, offset printing, anastatic printing or gravure printing; a soft lithography method such as a microcontact printing method; or a combination of two or more of these methods. Alternatively, it is possible to employ a method similar to application. Examples of the method similar to application include: a Langmuir-Blodgett method by which to (i) drop, onto the surface of water, a composition containing the organic semiconductor material to produce a monomolecular film of a semiconductor layer and (ii) transfer the monomolecular film to a substrate to form a stack of layers; and a method by which to sandwich a material in the form of liquid crystal or in the form of melt between two substrates to thereby introduce the material between the substrates using the capillary phenomenon. The film thickness of the organic semiconductor layer formed by the foregoing methods is preferably as small as possible, provided that the functions are not impaired. As the film thickness becomes large, a leakage current may increase. The film thickness of the organic semiconductor layer is the same as the foregoing film thickness of the semiconductor layer 4.

The semiconductor layer thus formed can be subjected to an aftertreatment so that its semiconducting properties are improved. For example, after the semiconductor layer is formed, the substrate can be subjected to a heat treatment. This reduces for example deformation of the layer which deformation occurred when the layer was formed, and thus makes it possible to control alignment and orientation in the layer. Accordingly, it is possible to improve the semiconducting properties and make the semiconductor layer stable, and further possible to reduce pinholes etc. The heat treatment can be carried out in any stage provided that the semiconductor layer has been formed. The temperature of the heat treatment is not particularly limited, but is normally room temperature to 150°C, and preferably 40°C to 120°C. The length of time for which the heat treatment is carried out is not particularly limited, but is normally 1 second to 24 hours, and is preferably 1 minute to 1 hour. A heat treatment under optimum conditions makes it possible to dramatically improve heat resistance in the subsequent stages. The heat treatment can be carried out in the atmosphere or in an inert atmosphere such as nitrogen or argon.

Another aftertreatment for the semiconductor layer is a method by which, for the purpose of increasing or reducing the density of carriers in the layer, to treat the semiconductor layer with an oxidizing or reducing gas such as oxygen or hydrogen or with an oxidizing or reducing liquid, to thereby induce a change in the characteristics by oxidation or reduction. That is, a method of adding a minute amount of elements, atomic groups, molecules or polymers to a semiconductor layer, thereby increasing or reducing the density of carriers in the semiconductor layer and thus changing the semiconducting properties such as electric conductivity, carrier polarity (p to n-type conversion), and Fermi level. In particular, this method is used generally for a semiconductor element formed with use of an inorganic material such as silicon. This method can be achieved by for example (i) bringing the semiconductor layer into contact with a gas such as oxygen or hydrogen, (ii) immersing the semiconductor layer into a solution containing an acid such as hydrochloric acid, sulfuric acid and/or sulfonic acid and/or a Lewis acid such as PF₅, AsF₅, and/or FeCl₃ etc., or (iii) electrochemically treating halogen atoms such as iodine or metal atoms such as sodium or potassium etc. Such doping does not have to be carried out after the semiconductor layer has been formed. The doping can be carried out by (i) adding a material for the doping into a material from which the semiconductor layer is to be formed by a vacuum deposition method and causing codeposition, (ii) mixing the material into an atmosphere in which the semiconductor layer is produced (i.e., a method of producing a semiconductor layer in the presence of a doping material), or (iii) causing a collision of ions with the semiconductor layer by accelerating the ions in a vacuum.

### (Formation of insulation layer)

The gate insulation layer 5, which is made from the foregoing insulating material etc., is formed on the semiconductor layer 4 (refer to Fig. 1). The gate insulation layer 5 can be formed by for example: an application method such as spin coating, spray coating, dip coating, casting, bar coating or blade coating; a printing method such as screen printing, offset printing or ink-jet; or a dry process method such as a vacuum deposition method, molecular beam epitaxy, an ion cluster beam method, an ion plating method, a sputtering method, an atmospheric pressure plasma method, or a CVD method. Alternatively, the gate insulation layer 5 can be formed by a sol-gel method or a method of forming an oxide film on a surface of a metal like forming anodized aluminum on aluminum.

The film thickness of the gate insulation layer 5 is preferably as small as possible, provided that the functions of the gate insulation layer 5 are not impaired. The film thickness is normally 0.1 nm to 100 µm, preferably 0.5 nm to 50 µm, and more preferably 5 nm to 10 µm.

### (Formation of gate electrode)

The gate electrode 6 can be formed in the same manner as in the source electrode 2 and the drain electrode 3. The film thickness of the gate electrode 6 depends on the material, but is normally 1 nm to 100 µm, preferably 0.5 nm to 10 µm, and more preferably 1 nm to 5 µm.

### (Protection layer)

Forming the protection layer 7 from the foregoing protection layer material is advantageous in that it is possible to reduce the effects of outside air to the minimum and to make electrical characteristics of the field-effect transistor stable (refer to Fig. 1). The film thickness of the protection layer 7 can be any film thickness depending on its purpose, but is normally 100 nm to 1 mm. The protection layer can be formed by various methods. In a case where the protection layer is made from resin, the protection layer is formed by for example (i) a method of applying a solution containing the resin and thereafter drying the solution to form a resin film or (ii) a method of applying resin monomers or depositing the resin monomers and thereafter polymerizing the resin monomers. After the protection layer is formed, the protection layer can be subjected to a crosslinking treatment. In a case where the protection layer is made from an inorganic substance, the protection layer is formed by for example (i) a vacuum process such as a sputtering method or a deposition method or (ii) an application printing process such as a sol-gel method. According to the field-effect transistor of the present invention, the protection layer can be provided not only on the surface of the semiconductor layer but also between other layers as needed. The protection layer(s) thus provided may serve to make the electrical characteristics of the field-effect transistor stable.

In general, the operating characteristics of a field-effect transistor depend on the carrier mobility and conductivity of a semiconductor layer, capacitance of an insulation layer, a configuration of an element (e.g., the distance between source and drain electrodes and the width of each of the electrodes, and the film thickness of an insulation layer) and the like. An organic material for use in the semiconductor layer of the field-effect transistor is required to have a high carrier mobility. In this regard, a compound represented by the formula (1) of the present invention, which compound can be produced at low cost, expresses a high carrier mobility required for an organic semiconductor material. Further, the field-effect transistor of the present invention can be produced by a relatively low-temperature process. Therefore, a flexible material such as a plastic plate or a plastic film etc., which is not usable at high temperatures, can be used as a substrate. This makes it possible to produce a lightweight, highly flexible element that is less prone to breakages, and such an element can be used as a switching element etc. in an active matrix of a display. Examples of the display include a liquid crystal display, a polymer dispersed liquid crystal display, an electrophoresis display, an EL display, an electrochromic display, and a particle rotation display. Further, the field-effect transistor of the present invention has good film-forming property, and thus can be produced by an application printing process such as application. As such, the present invention is applicable to a production of a field-effect transistor for use in a large-area display at remarkably low cost as compared to a conventional vacuum deposition process.

The field-effect transistor of the present invention is usable as a digital or analog element such as a memory circuit element, a signal driver circuit element or a signal processing circuit element. Combining these uses makes it possible to produce an IC card or an IC tag. Further, the field-effect transistor of the present invention is capable of changing its characteristics in response to an external stimulus such as that of a chemical substance, and thus shows promise of being usable as a sensor using a FET.

The present invention also encompasses the following configurations <2> to <16>.
<2> The organic semiconductor material according to <1>, wherein the compound represented by the formula (2) is a compound that has a molecular weight of 5000 or greater and is represented by the formula (3): 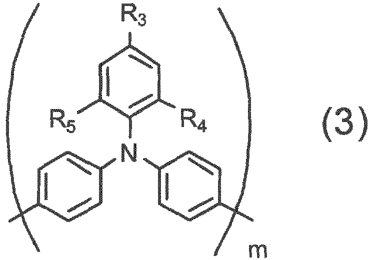 wherein at least one of R₃, R₄ and R₅ represents a halogen atom, a C 1-C4 alkyl group, a C1-C4 alkoxyl group, a C1-C4 halogeno alkyl group, a C1-C4 halogeno alkoxyl group or a cyano group; and the other(s) independently represent a hydrogen atom, a halogen atom, a C 1-C4 alkyl group, a C1-C4 alkoxyl group, a C1-C4 halogeno alkyl group, a C1-C4 halogeno alkoxyl group or a cyano group; and m represents an integer of 10 or greater.
<3> The organic semiconductor material according to <2>, wherein at least one of R₃, R₄ and R₅ in the formula (3) represents a methyl group, a trifluoromethyl group, a methoxy group, a trifluoromethoxy group or a fluoro group; and the other(s) represents a hydrogen atom, a methyl group, a trifluoromethyl group, a methoxy group, a trifluoromethoxy group or a fluoro group.
<4> The organic semiconductor material according to any one of <1> through <3>, wherein R₁ and R₂ in the formula (1) independently represent a linear C6-C12 alkyl group.
<5> The organic semiconductor material according to any one of <1> through <4>, wherein the ratio of the compound represented by the formula (1) to the compound represented by the formula (2) is 5 : 1 to 1 : 1.
<6> An organic semiconductor composition obtained by dissolving and/or dispersing an organic semiconductor material recited in any one of <1> through <5> in at least one organic solvent.
<7> The organic semiconductor composition according to <6>, comprising a solution that contains at least one organic solvent having a boiling point of 150°C or higher.
<8> The organic semiconductor composition according to <7>, comprising a solution that contains at least one organic solvent having a boiling point of 180°C or higher.
<9> The organic semiconductor composition according to any one of <6> through <8>, wherein the solid content of the organic semiconductor material is not less than 0.5% but not more than 5%.
<10> An organic thin film comprising an organic semiconductor material recited in any one of <1> through <5>.
<11> An organic thin film formed by an application printing process with use of an organic semiconductor composition recited in any one of <6> through <9>.
<12> A field-effect transistor comprising an organic semiconductor material recited in any one of <1> through <5>.
<13> The field-effect transistor according to <12>, which has a top-gate structure.
<14> The field-effect transistor according to <13>, which has a top-gate bottom-contact structure having a top-gate structure in which: a semiconductor layer containing the organic semiconductor material is provided on a substrate that has a source electrode and a drain electrode; a gate insulation layer is provided to part or all of an upper portion of the organic semiconductor material; and a gate electrode is provided so as to be in contact with an upper portion of the gate insulation layer.
<15> A method of producing a field-effect transistor, comprising forming a semiconductor layer by an application printing process with use of an organic semiconductor composition recited in any one of <6> through <9>.
<16> A method of producing a field-effect transistor having a top-gate bottom-contact structure, including: forming a semiconductor layer by an application printing process with use of an organic semiconductor composition recited in any one of <6> through <9>; and forming a gate insulation layer on an upper portion of the semiconductor layer by the application printing process.

### Examples

The following description discusses the present invention in further detail, on the basis of Examples. Note, however, that the present invention is not limited to these examples. In the examples, unless otherwise particularly specified, the term "part(s)" means "part(s) by mass" and "%" means "% by mass".

### Example 1

### (Preparation of solution)

A compound (11) shown in Table 1 was dissolved in tetrahydronaphthalene to obtain a 4% solution, and poly(bis (4-phenyl)2,4,6-trimethylphenylamine) (produced by Sigma-Aldrich) was dissolved in tetrahydronaphthalene to obtain a 4% solution. These solutions were mixed together at a ratio by mass of 1 : 1. In this way, a composition was prepared.

### (Production of transistor element)

A glass substrate on which source-drain patterns (gold electrodes: channel length 100 µm × channel width 15 mm, 36 patterns) were formed by photolithography was subjected to a plasma treatment. On the substrate, a 10 mM IPA solution of pentafluorobenzenethiol (produced by Aldrich) was applied by spin coating, and the substrate was subjected to an electrode SAM treatment. Next, a 10 mM toluene solution of phenylethyl trichlorosilane (produced by Aldrich) was applied to the substrate by spin coating, and the surface of the substrate was subjected to the SAM treatment. After that, the composition prepared as above from the compound (11) and poly(bis (4-phenyl)2,4,6-trimethylphenylamine) was applied by spin coating to form an organic thin film. Further, CYTOP (produced by Asahi Glass Co., Ltd.) was applied to the organic thin film by spin coating to form an organic insulation film. Then, gold was deposited on the organic insulation film by a vacuum deposition method using a metal mask, thereby forming a gate electrode. In this way, a top-gate bottom-contact element was produced.

### (Evaluation of characteristics)

The obtained organic field-effect transistor was evaluated for semiconducting properties of the 36 patterns on a single substrate, under the condition where the drain voltage was fixed at -2 V and the gate voltage Vg was changed from +20 V to -100 V. The mean value calculated from mobility in the 36 patterns was 1.6 cm²/Vs (maximum value was 2.0 cm²/Vs), and the standard deviation, which is an indicator of the dispersion within the substrate, was 0.24 cm²/Vs. Further, the average threshold voltage was -21 V, with the standard deviation of 1.9 V. That is, the organic field-effect transistor showed excellent mobility and uniformity in the substrate. The results not only suggest that the composition shows high mobility as compared to Non Patent Literature 2 in which the mobility is 0.53 cm²/Vs with the standard deviation of 0.24, but also suggest that the composition has uniform printing characteristics.

Further, as is clear from the semiconducting properties shown in Fig. 2, there was no hysteresis, and there was no change in the semiconducting properties when a voltage was repeatedly applied. In addition, as shown in Fig. 3, even after 139 days of exposure to the atmosphere, the mobility, the threshold voltage and the ON current changed only slightly. That is, excellent semiconducting properties were maintained.

### Comparative Example 1

### (Production of transistor element)

A top-gate bottom-contact element was produced in the same manner as in Example 1, except that the compound (11) was replaced with the following compound (101) described in Patent Literature 5.

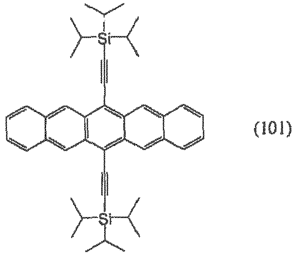

### (Evaluation of characteristics)

The obtained organic field-effect transistor was evaluated for semiconducting properties under the same conditions as above. The mean value calculated from mobility in the 36 patterns was 0.94 cm²/Vs, and the standard deviation was 0.24 cm²/Vs. These results were dramatically inferior to those of Example 1.

### Comparative Example 2

A transistor element was produced in the same manner as in Example 1, expect that the composition produced in Example 1 was replaced with a 3% tetrahydronaphthalene solution of the compound (11).

### (Evaluation of characteristics)

The obtained transistor element was evaluated for its semiconducting properties under the same conditions as in Example 1. The mean value calculated from mobility in the 36 patterns was as high as 2.75 cm²/Vs, whereas the standard deviation, which is an indicator of the dispersion within the substrate, was 0.85 cm²/Vs. That is, the dispersion in mobility in the electrodes was extremely large as compared to Example 1. In addition, in this element, a formed thin film had a crack, and many of the electrodes did not operate as transistor elements.

### Example 2

A transistor element was produced with use of the composition prepared in Example 1 in the same manner as in Example 1, except that the organic insulation film was changed from CYTOP to Teflon (registered trademark) AF1600 (produced by DuPont).

### (Evaluation of characteristics)

The obtained transistor element was evaluated for its semiconducting properties under the same conditions as in Example 1. As a result, the mean value calculated from mobility in the 36 patterns was 2.5 cm²/Vs (maximum value was 3.3 cm²/Vs), and the standard deviation, which is an indicator of the dispersion within the substrate, was 0.43 cm²/Vs. Further, the average threshold voltage was -15 V with the standard deviation of 2.8 V. That is, the transistor element showed excellent mobility and uniformity in the substrate.

### Example 3

A transistor element was produced in the same manner as in Example 2 except that poly(bis (4-phenyl)2,4,6-trimethylphenylamine) was replaced with poly(bis (4-phenyl)2,4-dimethyl phenylamine) (produced by HFR).

### (Evaluation of characteristics)

The transistor element thus obtained was evaluated for its semiconducting properties under the same conditions as in Example 1. As a result, the mean value calculated from mobility in the 36 patterns was 1.65 cm²/Vs (maximum value was 2.07 cm²/Vs), and the standard deviation, which is an indicator of the dispersion within the substrate, was 0.40 cm²/Vs. Further, the average threshold voltage was -17 V with the standard deviation of 2.2 V. That is, the transistor element showed excellent mobility and uniformity in the substrate.

### Example 4

The composition used in Example 3 was applied to a glass substrate by spin coating in the same manner as in Example 3, and thereafter heated at 120°C for 30 minutes. After that, CYTOP (produced by Asahi Glass Co., Ltd.) was applied to the obtained organic thin film by spin coating to form an organic insulation film. Then, gold was deposited on the organic insulation film by a vacuum deposition method using a metal mask, thereby forming a gate electrode. In this way, a top-gate bottom-contact element was produced.

### (Evaluation of characteristics)

The transistor element thus obtained was evaluated for its semiconducting properties under the same conditions as in Example 1. As a result, the mean value calculated from mobility in the 36 patterns was 1.98 cm²/Vs, and the standard deviation, which is an indicator of the dispersion within the substrate, was 0.33 cm²/Vs. Further, the average threshold voltage was -17 V with the standard deviation of 2.2 V. That is, the transistor element showed excellent mobility and uniformity in the substrate.

### (Heat resistance test)

This transistor element was further heated at 130°C for 30 minutes, and the heat resistance of the transistor element when exposed to high temperatures was tested. As a result, the mean value calculated from mobility in the 36 patterns was 2.16 cm²/Vs, and the standard deviation, which is an indicator of the dispersion within the substrate, was 0.12 cm²/Vs. Further, the average threshold voltage was -17 V with the standard deviation of 1.4 V. That is, no significant change was observed as compared to the characteristics before the heat resistance test.

### Example 5

A transistor element was produced in the same manner as in Example 1, except that poly(bis (4-phenyl)2,4,6-trimethylphenylamine) used in Example 1 was replaced with poly(bis (4-phenyl)4-fluorophenylamine) (produced by HFR).

### (Evaluation of characteristics)

The obtained transistor element was evaluated for its semiconducting properties under the same conditions as in Example 1. As a result, the mean value calculated from mobility in the 36 patterns was 1.82 cm²/Vs (maximum value was 2.21 cm²/Vs), and the standard deviation, which is an indicator of the dispersion within the substrate, was 0.39 cm²/Vs. Further, the average threshold voltage was -1.6 V with the standard deviation of 1.6 V. That is, the transistor element showed excellent mobility and uniformity in the substrate.

### Example 6

A transistor element was produced in the same manner as in Example 4, except that, in the composition used in Example 4, the ratio by mass of the compound (11) to poly(bis(4-phenyl) 4-fluorophenylamine) was changed from 1 : 1 to 3 : 1.

### (Evaluation of characteristics)

The obtained transistor element was evaluated for its semiconducting properties under the same conditions as in Example 1. As a result, the mean value calculated from mobility in the 36 patterns was 2.79 cm²/Vs, and the standard deviation, which is an indicator of the dispersion within the substrate, was 0.59 cm²/Vs. Further, the average threshold voltage was 0.85 V with the standard deviation of 0.7 V. That is, the transistor element showed excellent mobility and uniformity in the substrate.

### Example 7

A transistor element was produced in the same manner as in Example 4, except that, in the composition used in Example 4, the ratio by mass of the compound (11) to poly(bis(4-phenyl) 4-fluorophenylamine) was changed from 1 : 1 to 5 : 1.

### (Evaluation of characteristics)

The obtained transistor element was evaluated for its semiconducting properties under the same conditions as in Example 1. As a result, the mean value calculated from mobility in the 36 patterns was 2.33 cm²/Vs, and the standard deviation, which is an indicator of the dispersion within the substrate, was 0.45 cm²/Vs. Further, the average threshold voltage was 2.9 V with the standard deviation of 1.9 V. That is, the transistor element showed excellent mobility and uniformity in the substrate.

As has been described, the following was confirmed. That is, a field-effect transistor formed with use of an organic semiconductor material of the present invention (i) operates stably in the atmosphere, (ii) does not employ a vacuum deposition method that requires special equipment etc. when producing a semiconductor layer, (iii) does not require complicated operations such as patterning when carrying out a surface treatment of a substrate, and (iv) is possible to produce easily and at low cost by an application method etc. Further, the field-effect transistor formed with use of the organic semiconductor material of the present invention shows high semiconducting properties and uniformity as compared to conventionally known field-effect transistors such as a field-effect transistor formed with use of a pentacene derivative and a filed-effective transistor formed with use of only benzothieno [3,2-b][1] benzothiophene containing an alkyl group.

In addition, the following was confirmed. It has been known that, according to conventional organic field-effect transistors formed with use of a pentacene derivative etc., a compound used in a semiconductor layer decomposes when subjected to moisture in the atmosphere. That is, conventional organic field-effect transistors are not stable in the atmosphere. In contrast, a field-effect transistor of the present invention shows semiconducting properties that do not change significantly even in the measurement after 139 days, and thus is highly stable even in the atmosphere and is long life. In particular, a field-effect transistor having a top-gate bottom-contact structure not only provides more excellent transistor performance but also is highly durable.

### Reference Signs List

- 1: Substrate
- 2: Source electrode
- 3: Drain electrode
- 4: Semiconductor layer
- 5: Gate insulation layer
- 6: Gate electrode
- 7: Protection layer

## Claims

1. An organic semiconductor material comprising a compound represented by the formula (1) and a compound represented by the formula (2): 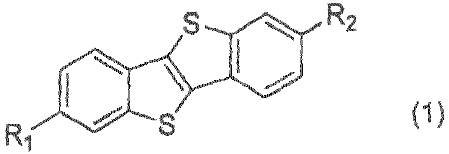 wherein R₁ and R₂ independently represent an unsubstituted or halogen-substituted C1-C36 aliphatic hydrocarbon group; and 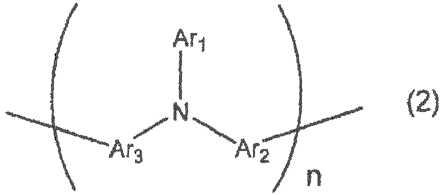
wherein Ar₁ represents a phenyl group substituted with a halogen atom, a C1-C12 alkyl group, a C1-C12 alkoxyl group, a C1-C12 halogeno alkyl group, a C1-C12 halogeno alkoxyl group or a cyano group,
Ar₂ and Ar₃ independently represent a phenyl group substituted with a hydrogen atom, a halogen atom, a C1-C12 alkyl group, a C1-C12 alkoxyl group, a C1-C12 halogeno alkyl group, a C1-C12 halogeno alkoxyl group or a cyano group, and n is an integer of 6 or greater, and
the compound represented by the formula (2) has a molecular weight of 5,000 or greater.

2. The organic semiconductor material according to claim 1, wherein:
the compound represented by the formula (2) is a compound that has a molecular weight of 5000 or greater and is represented by the formula (3): 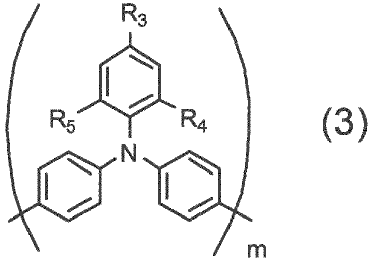 wherein at least one of R₃, R₄ and R₅ represents a halogen atom, a C1-C4 alkyl group, a C1-C4 alkoxyl group, a C1-C4 halogeno alkyl group, a C1-C4 halogeno alkoxyl group or a cyano group; and the other(s) independently represent a hydrogen atom, a halogen atom, a C1-C4 alkyl group, a C1-C4 alkoxyl group, a C1-C4 halogeno alkyl group, a C1-C4 halogeno alkoxyl group or a cyano group; and m represents an integer of 10 or greater.

3. The organic semiconductor material according to claim 2, wherein at least one of R₃, R₄ and R₅ in the formula (3) represents a methyl group, a trifluoromethyl group, a methoxy group, a trifluoromethoxy group or a fluoro group; and the other(s) represents a hydrogen atom, a methyl group, a trifluoromethyl group, a methoxy group, a trifluoromethoxy group or a fluoro group.

4. The organic semiconductor material according to any one of claims 1 through 3, wherein R₁ and R₂ in the formula (1) independently represent a linear C6-C12 alkyl group.

5. The organic semiconductor material according to any one of claims 1 through 4, wherein the ratio of the compound represented by the formula (1) to the compound represented by the formula (2) is 5 : 1 to 1 : 1.

6. An organic semiconductor composition obtained by dissolving and/or dispersing an organic semiconductor material recited in any one of claims 1 through 5 in at least one organic solvent.

7. The organic semiconductor composition according to claim 6, comprising a solution that contains at least one organic solvent having a boiling point of 150°C or higher.

8. The organic semiconductor composition according to claim 7, comprising a solution that contains at least one organic solvent having a boiling point of 180°C or higher.

9. The organic semiconductor composition according to any one of claims 6 through 8, wherein the solid content of the organic semiconductor material is not less than 0.5% but not more than 5%.

10. An organic thin film comprising an organic semiconductor material recited in any one of claims 1 through 5.

11. An organic thin film formed by an application printing process with use of an organic semiconductor composition recited in any one of claims 6 through 9.

12. A field-effect transistor comprising an organic semiconductor material recited in any one of claims 1 through 5.

13. The field-effect transistor according to claim 12, which has a top-gate structure.

14. The field-effect transistor according to claim 13, which has a top-gate bottom-contact structure having a top-gate structure in which:
a semiconductor layer containing the organic semiconductor material is provided on a substrate that has a source electrode and a drain electrode;
a gate insulation layer is provided to part or all of an upper portion of the organic semiconductor material; and
a gate electrode is provided so as to be in contact with an upper portion of the gate insulation layer.

15. A method of producing a field-effect transistor, comprising forming a semiconductor layer by an application printing process with use of an organic semiconductor composition recited in any one of claims 6 through 9.

16. A method, according to claim 15, of producing a field-effect transistor having a top-gate
bottom-contact structure, comprising:
forming a semiconductor layer by an application printing process with use of an organic semiconductor composition recited in any one of claims 6 through 9; and
forming a gate insulation layer on an upper portion of the semiconductor layer by the application printing process.

## Patentansprüche

1. Ein organisches Halbleitermaterial, welches eine durch Formel (1) und Formel (2) dargestellte Verbindung umfasst: 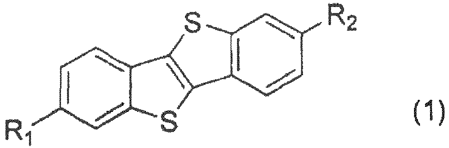 wobei R₁ und R₂ unabhängig voneinander eine unsubstituierte oder Halogensubstituierte C1-C36 aliphatische Kohlenwasserstoffgruppe darstellen; und 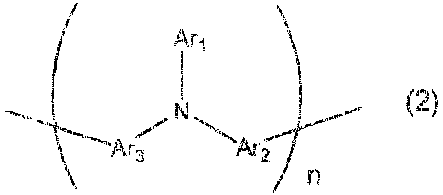
wobei Ar₁ eine mit einem Halogenatom, einer C1-C12 Alkylgruppe, einer C1-C12 Alkoxygruppe, einer C1-C12 Halogenoalkylgruppe, einer C1-C12 Halogenoalkoxygruppe oder einer Cyanogruppe substituierte Phenylgruppe darstellt,
Ar₂ und Ar₃ unabhängig voneinander eine mit einem Wasserstoffatom, einem Halogenatom, einer C1-C12 Alkylgruppe, einer C1-C12 Alkoxygruppe, einer C1-C12 Halogenoalkylgruppe, einer C1-C12 Halogenoalkoxygruppe oder einer Cyanogruppe substituierte Phenylgruppe darstellen, und n eine ganze Zahl von 6 oder größer ist, und
die Verbindung, welche durch Formel (2) dargestellt wird, ein Molekulargewicht von 5000 oder mehr hat.

2. Das organische Halbleitermaterial nach Anspruch 1, worin die Verbindung, welche durch Formel (2) dargestellt wird, ein Molekulargewicht von 5000 oder größer hat und durch die Formel (3) dargestellt wird: 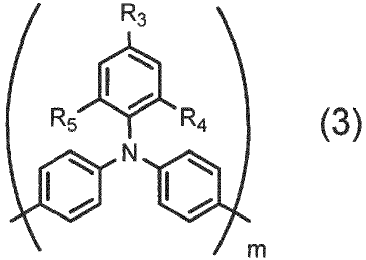 worin mindestens einer von R₃, R₄ und R₅ ein Halogenatom, eine C1-C4 Alkylgruppe, eine C1-C4 Alkoxygruppe, eine C1-C4 Halogenoalkylgruppe, eine C1-C4 Halogenoalkoxygruppe oder eine Cyanogruppe darstellt und die andere(n) unabhängig ein Wasserstoffatom, ein Halogenatom, eine C1-C4 Alkylgruppe, eine C1-C4 Alkoxygruppe, eine C1-C4 Halogenoalkylgruppe, eine C1-C4 Halogenoalkoxygruppe oder eine Cyanogruppe darstellt; und m eine ganze Zahl von 10 oder größer darstellt.

3. Das organische Halbleitermaterial nach Anspruch 2, worin mindestens einer von R₃, R₄ und R₅ in der Formel (3) eine Methylgruppe, eine Trifluoromethylgruppe, eine Methoxygruppe, eine Trifluoromethoxygruppe oder eine Fluorogruppe darstellt; und die andere(n) ein Wasserstoffatom, eine Methylgruppe, eine Trifluoromethylgruppe, eine Methoxygruppe, eine Trifluoromethoxygruppe oder eine Fluorogruppe darstellen.

4. Das organische Halbleitermaterial nach einem der Ansprüche 1 bis 3, worin R₁ und R₂ in der Formel (1) unabhängig voneinander eine lineare C6-C12 Alkylgruppe darstellen.

5. Das organische Halbleitermaterial nach einem der Ansprüche 1 bis 4, worin das Verhältnis der Verbindung welche von Formel (1) dargestellt wird zu der Verbindung welche von Formel (2) dargestellt wird 5 : 1 bis 1 : 1 beträgt.

6. Eine organische Halbleiterzusammensetzung, welche durch Lösen und/oder Dispergieren eines organischen Halbleitermaterials, gemäß einem der Ansprüche 1 bis 5, in mindestens einem organischen Lösungsmittel erhalten wird.

7. Die organische Halbleiterzusammensetzung nach Anspruch 6, welche eine Lösung umfasst, die mindestens ein organisches Lösungsmittel, welches einen Siedepunkt von 150°C oder höher hat, enthält.

8. Die organische Halbleiterzusammensetzung nach Anspruch 7, welche eine Lösung umfasst, die mindestens ein organisches Lösungsmittel mit einem Siedepunkt von 180°C oder höher enthält.

9. Die organische Halbleiterzusammensetzung nach einem der Ansprüche 6 bis 8, wobei der Feststoffanteil des organischen Halbleitermaterials mindestens 0,5% aber nicht mehr als 5% beträgt.

10. Eine organische Dünnschicht, welche ein organisches Halbleitermaterial nach einem der Ansprüche 1 bis 5 umfasst.

11. Eine organische Dünnschicht, welche durch ein Anwendungsdruckverfahren unter Verwendung einer organischen Halbleiterzusammensetzung gemäß einem der Ansprüche 6 bis 9 ausgebildet wird.

12. Ein Feldeffekttransistor, welcher ein organisches Halbleitermaterial gemäß einem der Ansprüche 1 bis 5 umfasst.

13. Der Feldeffekttransistor nach Anspruch 12, welcher eine top-gate Struktur hat.

14. Der Feldeffekttransistor nach Anspruch 13, welcher eine top-gate bottom-contact Struktur hat, wobei in der top-gate Struktur:
eine Halbleiterschicht, welche das organische Halbleitermaterial enthält, auf einem Substrat bereitgestellt ist, welches eine Quell-Elektrode und eine Drain-Elektrode besitzt;
eine Gate-Isolationsschicht für einen Teil oder den gesamten oberen Bereich des organischen Halbleitermaterials bereitgestellt ist; und
eine Gate Elektrode so bereitgestellt wird, dass sie in Kontakt mit einem oberen Bereich der Gate-Isolationsschicht ist.

15. Ein Verfahren zur Herstellung eines Feldeffekttransistors, welches das Ausbilden einer Halbleiterschicht durch ein Anwendungsdruckverfahren unter Verwendung einer organischen Halbleiterzusammensetzung nach einem der Ansprüche 6 bis 9 umfasst.

16. Ein Verfahren nach Anspruch 15 zur Herstellung eines Feldeffekttransistors mit einer top-gate bottom-contact Struktur, welches die folgenden Schritte umfasst:
Ausbildung einer Halbleiterschicht durch ein Anwendungsdruckverfahren unter Verwendung einer organischen Halbleiterzusammensetzung nach einem der Ansprüche 6 bis 9; und
Ausbildung einer Gate-Isolationsschicht auf einem oberen Bereich der Halbleiterschicht durch das Anwendungsdruckverfahren.

## Revendications

1. Matériau semi-conducteur organique comprenant un composé représenté par la formule (1) et un composé représenté par la formule (2) : 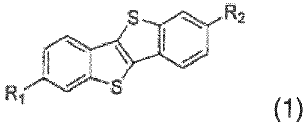 où R₁ et R₂ représentent indépendamment un groupe hydrocarboné aliphatique en C1 à C36 non substitué ou substitué par un halogène ; et 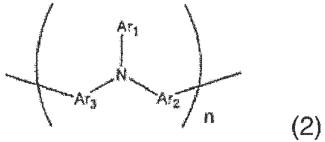
où Ar₁ représente un groupe phényle substitué par un atome d'halogène, un groupe alkyle en C1 à C12, un groupe alcoxyle en C1 à C12, un groupe halogénoalkyle en C1 à C12, un groupe halogénoalcoxyle en C1 à C12 ou un groupe cyano,
Ar₂ et Ar₃ représentent indépendamment un groupe phényle substitué par un atome d'hydrogène, un atome d'halogène, un groupe alkyle en C1 à C12, un groupe alcoxyle en C1 à C12, un groupe halogénoalkyle en C1 à C12, un groupe halogénoalcoxyle en C1 à C12 ou un groupe cyano, et n représente un nombre entier supérieur ou égal à 6, et
le composé représenté par la formule (2) a un poids moléculaire supérieur ou égal à 5000.

2. Matériau organique semi-conducteur selon la revendication 1, dans lequel :
le composé représenté par la formule (2) est un composé qui a un poids moléculaire supérieur ou égal à 5000 et est représenté par la formule (3) : 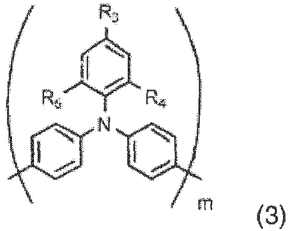 où au moins l'un parmi R₃, R₄ et R₅ représente un atome d'halogène, un groupe alkyle en C1 à C4, un groupe alcoxyle en C1 à C4, un groupe halogénoalkyle en C1 à C4, un groupe halogénoalcoxyle en C1 à C4 ou un groupe cyano ; et l'autre/les autres représente/représentent indépendamment un atome d'hydrogène, un atome d'halogène, un groupe alkyle en C1 à C4, un groupe alcoxyle en C1 à C4, un groupe halogénoalkyle en C1 à C4, un groupe halogénoalcoxyle en C1 à C4 ou un groupe cyano ; et m représente un nombre entier supérieur ou égal à 10.

3. Matériau semi-conducteur organique selon la revendication 2, dans lequel au moins l'un parmi R₃, R₄ et R₅ dans la formule (3) représente un groupe méthyle, un groupe trifluorométhyle, un groupe méthoxy, un groupe trifluorométhoxy ou un groupe fluoro ; et l'autre/les autres représente/représentent un atome d'hydrogène, un groupe méthyle, un groupe trifluorométhyle, un groupe méthoxy, un groupe trifluorométhoxy ou un groupe fluoro.

4. Matériau semi-conducteur organique selon l'une quelconque des revendications 1 à 3, dans lequel R₁ et R₂ dans la formule (1) représentent indépendamment un groupe alkyle linéaire en C6 à C12.

5. Matériau semi-conducteur organique selon l'une quelconque des revendications 1 à 4, dans lequel le rapport du composé représenté par la formule (1) sur le composé représenté par la formule (2) est compris entre 5:1 et 1:1.

6. Composition semi-conductrice organique obtenue par dissolution et/ou dispersion d'un matériau semi-conducteur organique selon l'une quelconque des revendications 1 à 5 dans au moins un solvant organique.

7. Composition semi-conductrice organique selon la revendication 6, comprenant une solution qui contient au moins un solvant organique ayant un point d'ébullition supérieur ou égal à 150°C.

8. Composition semi-conductrice organique selon la revendication 7, comprenant une solution qui contient au moins un solvant organique ayant un point d'ébullition supérieur ou égal à 180°C.

9. Composition semi-conductrice organique selon l'une quelconque des revendications 6 à 8, dans laquelle la teneur en matières solides du matériau semi-conducteur organique est supérieure ou égale à 0,5% mais inférieure ou égale à 5%.

10. Film mince organique comprenant un matériau semi-conducteur organique selon l'une quelconque des revendications 1 à 5.

11. Film mince organique formé par un procédé d'impression directe en utilisant une composition semi-conductrice organique selon l'une quelconque des revendications 6 à 9.

12. Transistor à effet de champ comprenant un matériau semi-conducteur organique selon l'une quelconque des revendications 1 à 5.

13. Transistor à effet de champ selon la revendication 12, qui a une structure de grille supérieure.

14. Transistor à effet de champ selon la revendication 13, qui a une structure de contact inférieur à grille supérieure ayant une structure de grille supérieure dans laquelle :
une couche semi-conductrice contenant le matériau semi-conducteur organique est prévue sur un substrat qui a une électrode de source et une électrode de drain ;
une couche d'isolation de grille est prévue sur une partie ou sur la totalité d'une partie supérieure du matériau semi-conducteur organique ; et
une électrode de grille est prévue de manière à être en contact avec une partie supérieure de la couche d'isolation de grille.

15. Procédé de production d'un transistor à effet de champ, comprenant le fait de former une couche semi-conductrice par un procédé d'impression directe en utilisant une composition semi-conductrice organique selon l'une quelconque des revendications 6 à 9.

16. Procédé, selon la revendication 15, de production d'un transistor à effet de champ ayant une structure de contact inférieur à grille supérieure, comprenant le fait :
de former une couche semi-conductrice par un procédé d'impression directe en utilisant une composition semi-conductrice organique selon l'une quelconque des revendications 6 à 9 ; et
de former une couche d'isolation de grille sur une partie supérieure de la couche semi-conductrice par le procédé d'impression directe.
